# EUROPEAN PATENT APPLICATION

(11) **EP 4 276 169 A1**
(43) Date of publication of application: **15.11.2023**
(21) Application number: 23171514.5
(22) Date of filing: 04.05.2023
(51) Int. Cl.: C12M 1/26

(54) **SUCTION TIP AND BIOLOGICAL SUBJECT TRANSFER DEVICE**

(30) Priority: 12.05.2022 JP 2022079001
(71) Applicant: Yamaha Hatsudoki Kabushiki Kaisha, Iwata-shi, Shizuoka 438-8501 (JP)
(72) Inventor: ITO, Saburo, Iwata-shi, 438-8501 (JP)
(74) Representative: Müller-Boré & Partner Patentanwälte PartG mbB

(57) **Abstract**

A suction tip that sucks a biological subject includes a base tip and a sub-tip. The base tip includes a distal end portion having a distal end opening, and a tubular passage connected to the distal end opening. The sub-tip includes a suction port that sucks the biological subject, and a guide passage having one end connected to the suction port and the other end that receives the distal end portion of the base tip. The base tip and the sub-tip are coupled and integrated by externally fitting the other end of the guide passage to the distal end portion, the integration forming one suction path in which the tubular passage and the guide passage communicate with each other. The suction port has a size smaller than a size of the distal end opening.

## Description

### FIELD OF INVENTION

The present invention relates to a suction tip that sucks a biological subject, and a biological subject transfer device using the suction tip.

### BACKGROUND ART

For example, in the fields of medical and biological researches, work of moving cells may be performed from a culture container for culturing a biological subject (which may be referred to simply as a "cell" in the present specification) such as a single cell or a cell colony to a work container for performing inspection, observation, or the like. For this work, there is a known cell transfer device that performs operation of picking and holding required cells from the culture container by a head to which a suction tip is attached, and releasing the held cells to the work container.

As the above suction tip, JP 2007-315793A discloses a combined type suction tip formed by inserting a glass tube having a small diameter into a distal end opening of a tip main body. According to this suction tip, a space between an outer periphery of the inserted tube and the tip main body is used as a storage portion of a liquid overflowed by suction operation.

For example, in order to selectively suck a microcell having a size on the order of 10 µm such as a single cell, it is necessary to mold a distal end portion of the suction tip into a corresponding microsize. It is very difficult to manufacture a suction tip in which a proximal end portion has a size that enables attachment to a head while a distal end portion has the above-described microsize. For example, even when manufacturing is attempted in the form of a combined type suction tip as disclosed in JP 2007-315793A, it is difficult to position and insert the above glass tube having the corresponding microsize into the distal end opening of the tip main body without being broken.

### SUMMARY OF THE INVENTION

An object of the present invention is to provide a suction tip suitable for sucking a microsized biological subject and easy to manufacture, and a biological subject transfer device using the suction tip.

A suction tip according to one aspect of the present invention is a suction tip that sucks a biological subject, the suction tip including: a base tip including a distal end portion having a distal end opening, and a tubular passage connected to the distal end opening; and a sub-tip including a suction port that sucks the biological subject, and a guide passage having one end connected to the suction port and another end that receives the distal end portion of the base tip; in which the base tip and the sub-tip are coupled and integrated by externally fitting the other end of the guide passage to the distal end portion, the integration forming one suction path in which the tubular passage and the guide passage communicate with each other, and the suction port has a size smaller than a size of the distal end opening.

A biological subject transfer device according to another aspect of the present invention includes: a head to which the above suction tip is attached and which includes a mechanism that generates a suction force and a discharge force at the suction port; and a transfer mechanism that moves the head horizontally and moves the head up and down.

### BRIEF DESCRIPTION OF THE DRAWINGS

Fig. 1 is a perspective view illustrating an appearance of a suction tip according to an embodiment of the present invention;
Fig. 2 is a cross-sectional view of a base tip of the suction tip illustrated in Fig. 1;
Fig. 3 is a cross-sectional view of a sub-tip of the suction tip;
Fig. 4 is a cross-sectional view of a main part of the suction tip;
Fig. 5 is a view showing cell transfer steps A to D using the suction tip;
Figs. 6A to 6C are cross-sectional views illustrating an example of a procedure of externally fitting the sub-tip to the base tip;
Fig. 7 is a perspective view illustrating an example of a cell transfer line assembled to a cell transfer device;
Fig. 8 is a cross-sectional view illustrating an example of attaching a plunger and a head to the suction tip; and
Fig. 9 is a block diagram showing an electrical configuration of the cell transfer device.

### DETAILED DESCRIPTION

In the following, an embodiment of the present invention will be described in detail with reference to the accompanying drawings. A suction tip and a biological subject transfer device according to the present invention allow biological subjects such as various biologically derived cells, bacteria, and chromosomes to be considered subjects to be sucked and moved. In particular, the present invention is suitable for movement of a biological subject having a size on the order of 10 µm. Examples of biologically derived cells include single cells such as hemocyte cells and singulated cells, small tissue pieces such as Histoculture, cell aggregates such as spheroids and organoids, individuals such as zebrafish, nematodes, and fertilized eggs, and 2D or 3D cell colonies.

### [Structure of Suction Tip]

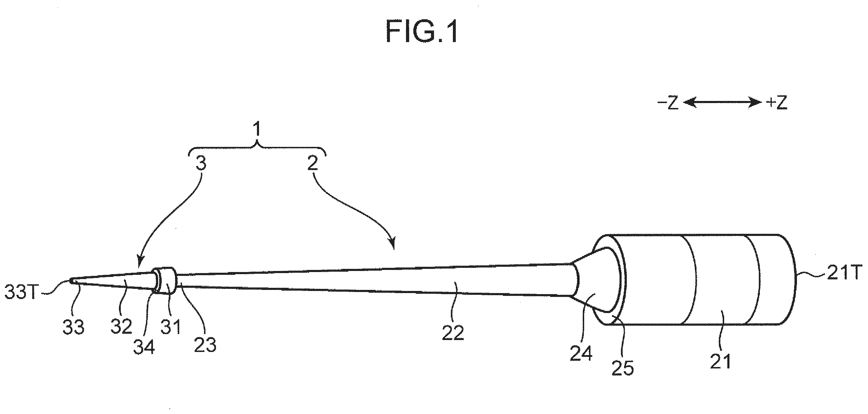

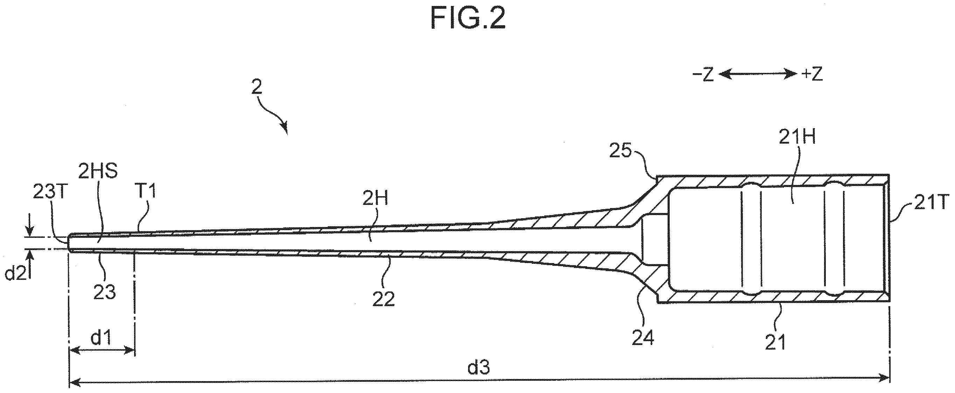

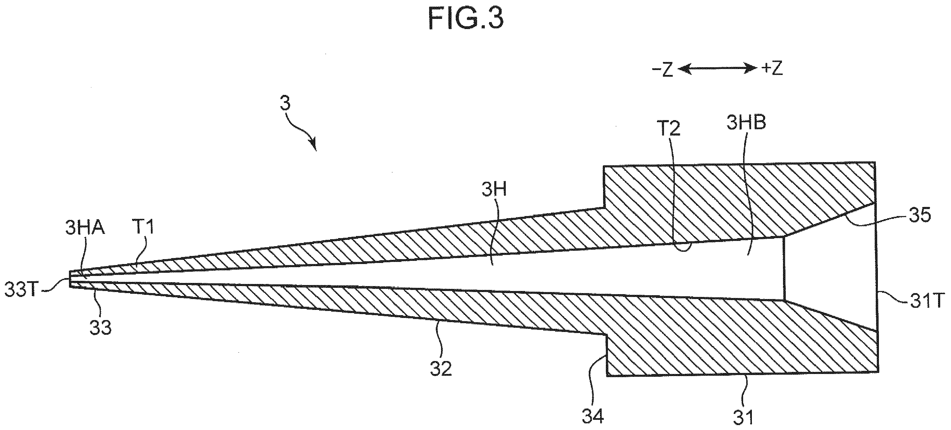

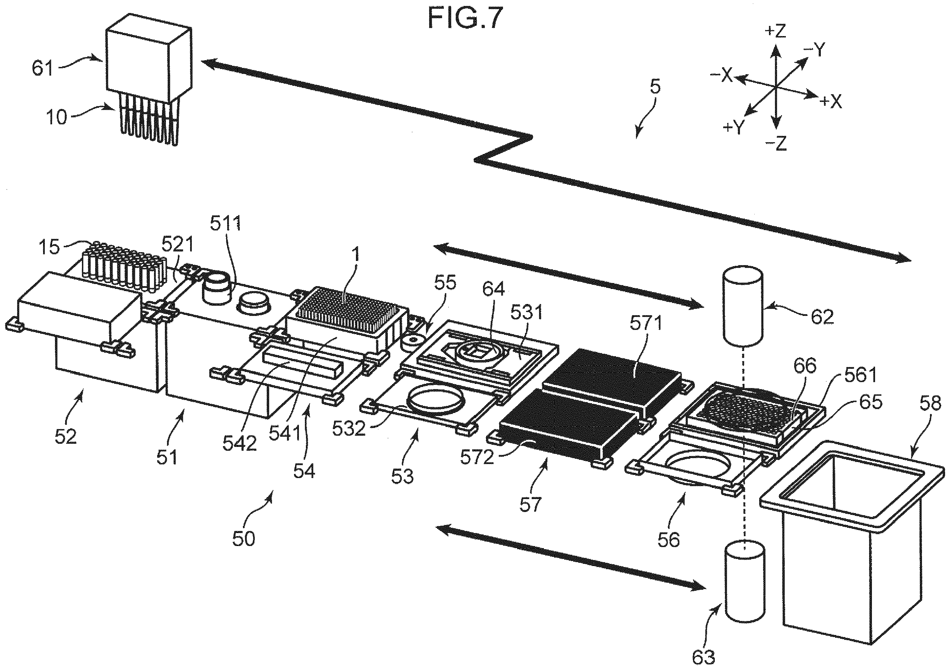

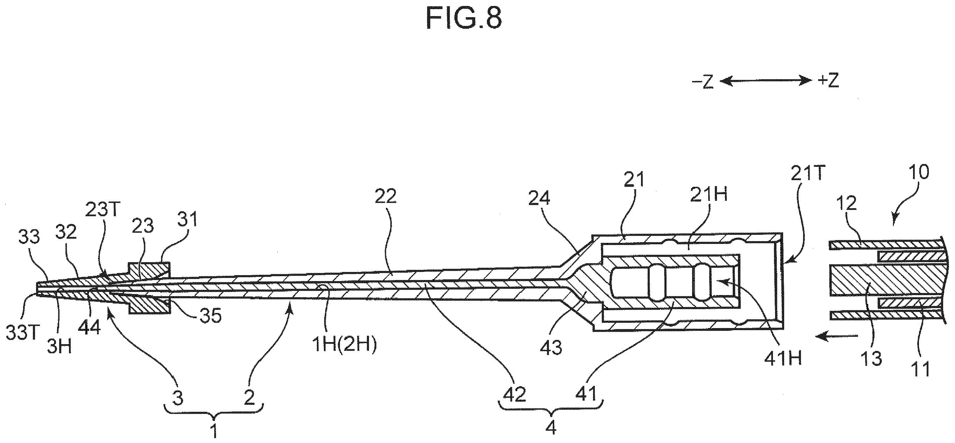

Fig. 1 is a perspective view illustrating an appearance of a suction tip 1 according to the embodiment of the present invention. The suction tip 1 is a tip capable of sucking and discharging the above-described biological subject, and is configured with a coupling body of a base tip 2 and a sub-tip 3. Fig. 2 is a cross-sectional view of the base tip 2, and Fig. 3 is a cross-sectional view of the sub-tip 3. In these drawings, direction indications of +Z and -Z corresponding to an up-down direction are applied so as to match direction indications in Figs. 7 and 8 to be described later.

Constituent materials of the base tip 2 and the sub-tip 3 are not particularly limited, and for example, resin, glass, ceramic, metal, or the like can be used as the constituent material. Among them, it is desirable to use a resin material having predetermined elasticity for both the base tip 2 and the sub-tip 3 from the viewpoint of suppressing damage at the time of assembling the suction tip 1 or at the time of suction operation. In this case, the base tip 2 and the sub-tip 3 can be manufactured by resin molding using a mold and a core.

The resin material may contain an appropriate additive. For example, a pigment may be included in the resin material so as to have a color that makes contrast be liable to stand out when an image of the suction tip 1 is captured by a camera. In addition, it is desirable to select a resin material having no toxicity to a cell to be sucked. Examples of the resin material may include polypropylene and polystyrene. The base tip 2 and the sub-tip 3 may be made of resin materials of the same material or resin materials of different materials. Using resin materials of the same material makes it possible to improve affinity between the base tip 2 and the sub-tip 3. In addition, it is easy to make molding conditions, toxicity evaluation, and the like of both the tips be uniform, which can contribute to facilitation of manufacture of the suction tip 1 . The base tip 2 and the sub-tip 3 may have the same hardness or different hardness. For example, the sub-tip 3 may be made of a resin material softer than that of the base tip 2.

### <Base Tip>

The base tip 2 includes a first proximal end portion 21, a first main body portion 22, and a first distal end portion 23 (insertion fitting part). The first proximal end portion 21 is formed of a large-diameter cylindrical body having a hollow portion 21H therein. The first main body portion 22 is connected to the first proximal end portion 21 on a -Z side (left side in Fig. 2) via a tapered cylinder portion 24, and a first proximal end opening 21T which is an opening end of the cylindrical body is provided on a +Z side. An end portion of a head 10 to be described later is inserted into the first proximal end opening 21T (Fig. 8).

The first main body portion 22 is formed of a cylindrical body having a small diameter and a long length as compared with the first proximal end portion 21. The first distal end portion 23 is a distal end portion having a circular cross section located on the -Z side of the first main body portion 22, and has a circular distal end opening 23T. The first distal end portion 23 is a region of the first main body portion 22 where the sub-tip 3 is externally fitted. Inside the first main body portion 22, a tubular passage 2H is formed whose -Z side is connected to the distal end opening 23T and whose +Z side is connected to the first proximal end opening 21T.

An outer peripheral surface of the first main body portion 22 is a first tapered surface T1 whose outer diameter decreases toward the distal end opening 23T. The tubular passage 2H also has a tapered surface whose inner diameter decreases toward the distal end opening 23T except for a region of the first distal end portion 23. Taper angles of the first main body portion 22 and the tubular passage 2H are gentle angles approximate to a draft angle of a mold. The outer peripheral surface of the first main body portion 22 may be a surface having no taper, or the first tapered surface T1 may be provided only on an outer peripheral surface of the first distal end portion 23. Fig. 2 illustrates an example in which the first main body portion 22 has a thickness which is large at the proximal end portion on the +Z side and becomes smaller toward the distal end opening 23T. Instead of this illustrated example, the thickness of the first main body portion 22 may be constant.

The tapered cylinder portion 24 is a part connecting the first proximal end portion 21 and the first main body portion 22, and has a tapered shape in which an outer diameter decreases toward the -Z side so as to fill a diameter difference between the first proximal end portion 21 and the first main body portion 22. A first flange part 25 (second stopper part) is provided at a +Z-side end portion of the tapered cylinder portion 24. The first flange part 25 is an annular flat surface extending in a direction orthogonal to the Z direction and provided to enable positioning in the Z direction which is an extending direction of the tubular passage 2H.

A distal end straight portion 2HS having a constant inner diameter is provided in the region of the first distal end portion 23 in the tubular passage 2H. The distal end straight portion 2HS is a part resultantly manufactured because at the time of resin molding of the base tip 2, for positioning a core for use in forming the tubular passage 2H, a region having a constant outer diameter is required at a front end of the core. As illustrated in Fig. 2, when an axial length of the distal end straight portion 2HS is denoted by d1 and an inner diameter of the tubular passage 2H at the distal end straight portion 2HS is denoted by d2, d1/d2 is referred to as an aspect ratio here. The aspect ratio of the distal end straight portion 2HS is desirably within a range in which the base tip 2 can be manufactured by resin molding. When the total length of the base tip 2 becomes longer with respect to the distal end straight portion 2HS, it is difficult to increase the aspect ratio of the distal end straight portion 2HS due to problems of workability of a mold and fluidity at the time of resin injection. For example, when a length d3 of the base tip 2 is about 20 mm to 40 mm and the inner diameter d2 of the distal end opening 23T is about 0.1 mm to 0.3 mm, the aspect ratio of the distal end straight portion 2HS can be selected from a range of 1.5 to 4, and in particular, is desirably selected from a range of 2 to 3. Note that in the present embodiment, the suction tip 1 can be provided with a suction port having a smaller diameter than the distal end opening 23T by coupling of the sub-tip 3 without setting the aspect ratio of the base tip 2 to be large.

### <Sub-tip>

The sub-tip 3 includes a second proximal end portion 31, a second main body portion 32, and a second distal end portion 33. The second proximal end portion 31 is an end portion region on the +Z side of the sub-tip 3 and is a cylindrical portion having a relatively large diameter. The second main body portion 32 is continuously provided on the -Z side of the second proximal end portion 31. The second main body portion 32 has a conical outer shape having a smaller diameter toward the -Z side. The second distal end portion 33 is a distal end portion having a circular cross section located on the -Z side of the second main body portion 32, and has a circular suction port 33T. The suction port 33T is an opening for sucking a cell in the suction tip 1 and discharging the sucked cell.

The suction port 33T has a size smaller than the distal end opening 23T of the base tip 2. In other words, the suction port 33T has a bore smaller than a bore of the distal end opening 23T. In addition, the second distal end portion 33 has an outer diameter smaller than an outer diameter of the first distal end portion 23 of the base tip 2. Specifically, coupling the sub-tip 3 allows the suction tip 1 to include the second distal end portion 33 having the small outer diameter and the suction port 33T having the small bore, which are difficult to manufacture by the base tip 2 alone.

A guide passage 3H is provided inside the sub-tip 3. The guide passage 3H has one end 3HA on the -Z side connected to the suction port 33T and the other end 3HB on the +Z side that receives the first distal end portion 23 of the base tip 2. An inner peripheral surface of the guide passage 3H is a second tapered surface T2 whose inner diameter decreases toward the suction port 33T. Although Fig. 3 illustrates an example in which a thickness of the second main body portion 32 becomes smaller toward the suction port 33T, the thickness may be constant. In addition, the guide passage 3H may not be tapered, and may be a passage having a uniform inner diameter.

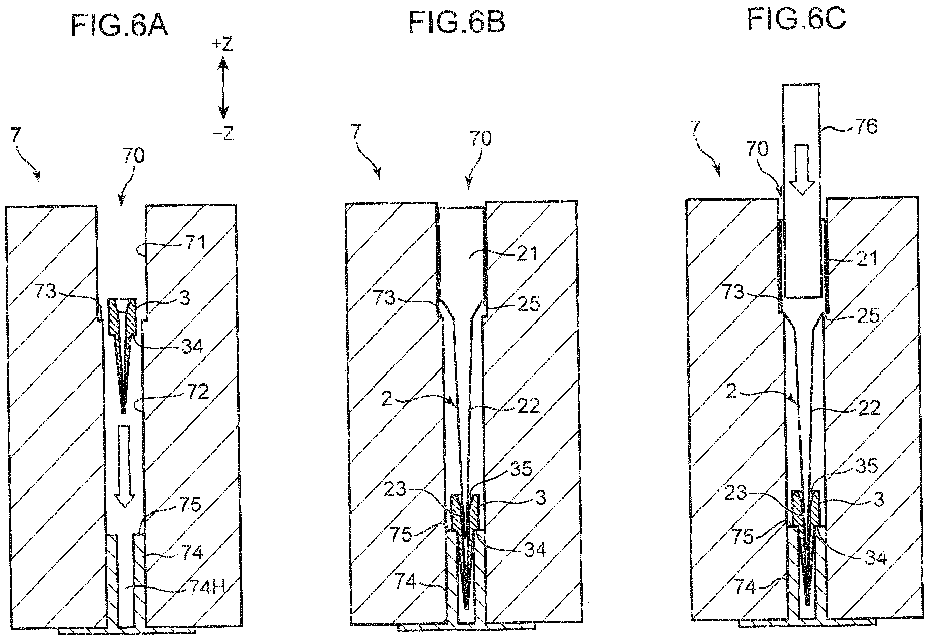

A second flange part 34 (first stopper part) is provided at a boundary between the second proximal end portion 31 and the second main body portion 32. The second flange part 34 is an annular flat surface extending in the direction orthogonal to the Z direction and provided to enable positioning in the Z direction which is an extending direction of the guide passage 3H. Roles of the first flange part 25 and the second flange part 34 will be described later with reference to Fig. 6.

At the other end 3HB of the guide passage 3H, a leading tapered surface 35 is continuously provided that leads the first distal end portion 23 to the other end 3HB when the sub-tip 3 is externally fitted to the base tip 2. The leading tapered surface 35 is configured by a tapered surface that further increases in diameter from the other end 3HB having the largest inner diameter in the guide passage 3H toward +Z. The leading tapered surface 35 is provided to guide the first distal end portion 23 of the base tip 2 at the time of the external fitting, thereby facilitating the coupling between the base tip 2 and the sub-tip 3. For example, when a mechanism capable of precisely aligning the base tip 2 and the sub-tip 3 on the Z-axis can be separately used, the sub-tip 3 may be configured without the leading tapered surface 35.

### [Coupling Structure between Base Tip and Sub-tip]

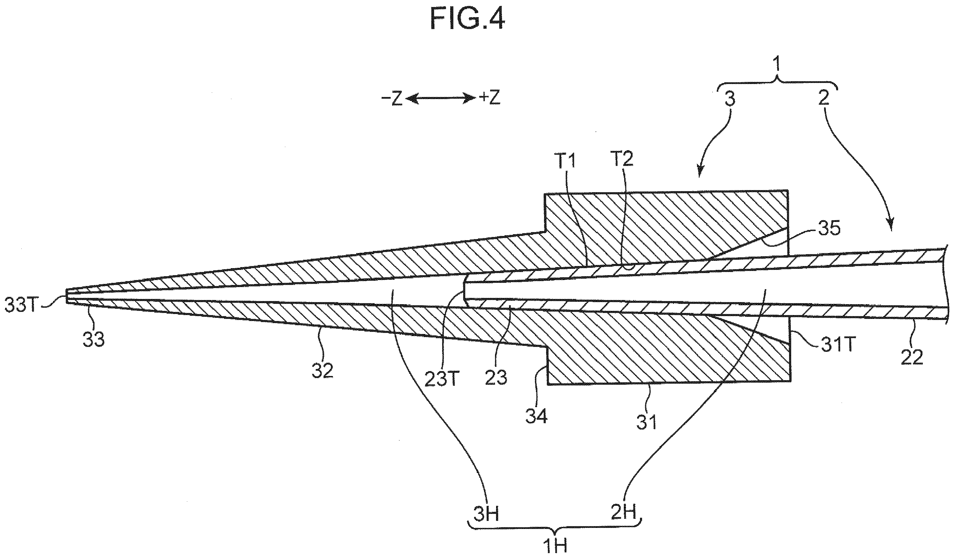

Fig. 4 is a cross-sectional view of a main part of the suction tip 1, the view illustrating an external fitting portion of the sub-tip 3 to the base tip 2. The base tip 2 and the sub-tip 3 are coupled and integrated by external fitting of the other end 3HB of the guide passage 3H to the first distal end portion 23. In other words, the first distal end portion 23 of the base tip 2 is inserted into the guide passage 3H from the other end 3HB, whereby the base tip 2 and the sub-tip 3 are integrated. Since the leading tapered surface 35 exists, the first distal end portion 23 can easily enter the guide passage 3H even if slight misalignment occurs between the base tip 2 and the sub-tip 3 at the time of the insertion.

The above coupling and integration forms one suction path 1H in which the tubular passage 2H of the base tip 2 and the guide passage 3H of the sub-tip 3 communicate with each other. Specifically, the distal end opening 23T of the base tip 2 faces a space in the guide passage 3H, so that the tubular passage 2H and the guide passage 3H communicate with each other. Inner peripheral surfaces of the tubular passage 2H and the guide passage 3H may be subjected to water repellent treatment so as to have water repellency to sucked liquid.

In a state where both the tips 2 and 3 are coupled and integrated, the outer peripheral surface of the first distal end portion 23 is in close contact with an inner peripheral surface of the other end 3HB of the guide passage 3H. In other words, interfaces between the first tapered surface T1 and the second tapered surface T2 come into close contact with each other. This brings the interfaces to have sealing properties. Accordingly, when a negative pressure or a positive pressure is applied to the hollow portion 21H located at the +Z end of the base tip 2 in the above coupled and integrated state, a negative pressure or a positive pressure can be generated at the suction port 33T through the suction path 1H. In order to improve the sealing properties, it is desirable that the first tapered surface T1 and the second tapered surface T2 are tapered surfaces having the same or similar tapered angle.

A bonding agent such as an adhesive may or may not be interposed on a fitting surface between the outer peripheral surface of the first distal end portion 23 and the inner peripheral surface of the guide passage 3H. Note that it is preferable to make the first distal end portion 23 be an insertion fitting part that is simply inserted into the other end 3HB of the guide passage 3H without interposing the adhesive because the base tip 2 and the sub-tip 3 can be coupled and integrated with each other, and a detaching step can be simplified. Forming the base tip 2 and the sub-tip 3 with a resin material having appropriate elasticity enables a coupling force of the integration to be secured by elastic deformation forces of the tips. The fitting surface may be fused by heat treatment, ultrasonic treatment, or the like.

As described above, the suction tip 1 of the present embodiment is a two-split type suction tip configured by a combination of the base tip 2 and the sub-tip 3. It is difficult to manufacture, with a single member, a suction tip having a very large aspect ratio, in which a distal end portion having a suction port is extremely small with respect to a tip length. In the case of the two-split type suction tip 1 as in the present embodiment, however, the base tip 2 and the sub-tip 3 can be individually manufactured within a range in which the aspect ratio does not become excessive, and can be coupled to each other to obtain one suction tip 1, resulting in facilitating manufacture of the tip. In other words, it is easy to reduce a size of the suction port 33T that sucks a cell.

In addition, it is structured such that the other end 3HB of the guide passage 3H of the sub-tip 3 is externally fit to the first distal end portion 23 of the base tip 2. Therefore, even if the second distal end portion 33 connected to the suction port 33T is set to have a minimum size, the sub-tip 3 is allowed to have the second proximal end portion 31 having a suitable size. In a mode in which a tube corresponding to a sub-tip is inserted into the distal end opening 23T of the base tip 2 as in the suction tip of JP 2007-315793 A, when miniaturization of the tube is required, work of the insertion is very difficult. However, according to the present embodiment in which the sub-tip 3 is externally fitted to the base tip 2, it is possible to facilitate assembly work for coupling and integrating both the tips.

Furthermore, by making the size of the suction port 33T of the sub-tip 3 smaller than a size of the distal end opening 23T of the base tip 2, it is possible to selectively suck a microcell with high accuracy. For example, it is possible to suck a target cell from a cell group randomly existing on a bottom surface of a dish into the suction tip 1 with good selectivity with respect to surrounding cells existing around the target cell. In addition, it is possible to make it difficult for the second distal end portion 33 to damage the surrounding cells during the suction operation.

An example of a size in a case where a microcell on the order of 10 µm is to be sucked will be described. The outer diameter of the first distal end portion 23 of the base tip 2 is set to 0.3 mm, the inner diameter d2 of the distal end opening 23T is set to 0.18 mm, the length d3 of the base tip 2 in the Z direction is set to 30 mm, and the length d1 of the distal end straight portion 2HS in the Z direction is set to 0.5 mm. An aspect ratio d1/d2 of the distal end straight portion 2HS is 0.5/0.18 = 2.8. The outer diameter of the second distal end portion 33 of the sub-tip 3 is set to 0.1 mm, an inner diameter of the suction port 33T is set to 0.05 mm, and a length of the sub-tip 3 in the Z direction is set to 9.0 mm. It is difficult to mold, with a single member, a suction tip with d3 on the order of 17.5 mm and an outer diameter of a distal end portion on the order of 0.1 mm. In the case of the two-split type suction tip 1 as in the present embodiment, however, the outer diameter of the second distal end portion 33 in which the suction port 33T is opened can be set to be on the order of 0.1 mm.

### [Usage Example of Suction Tip]

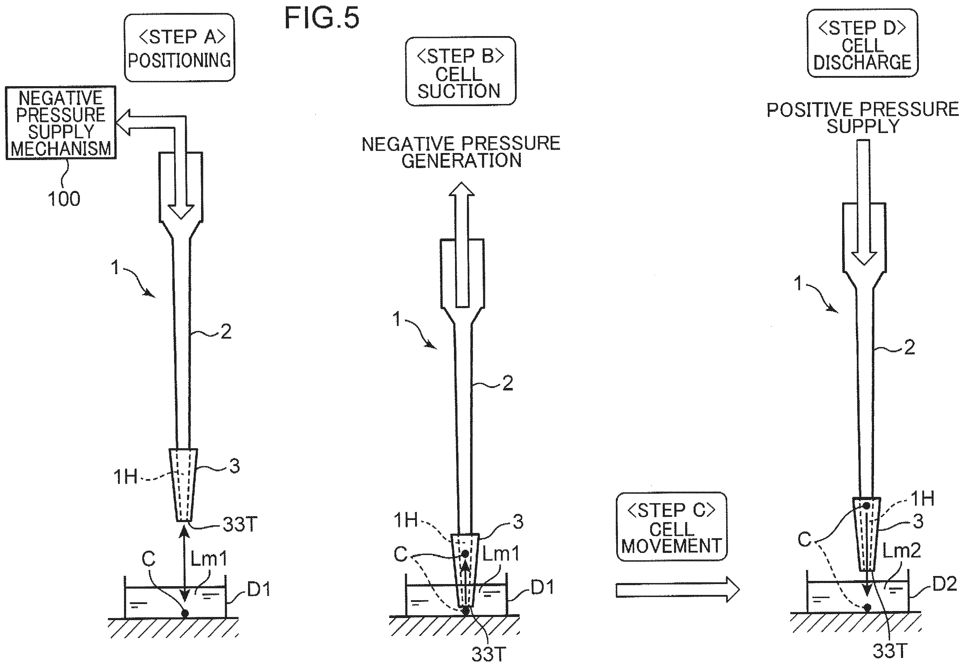

Next, a usage example of the above-described suction tip 1 will be described. Fig. 5 is a view showing cell movement steps A to D using the suction tip 1. A negative pressure supply mechanism 100 is attached to the suction tip 1. The negative pressure supply mechanism 100 is a mechanism that generates a negative pressure or a positive pressure at the suction port 33T by supplying a negative pressure or a positive pressure. As the negative pressure supply mechanism 100, a piston mechanism using a suction pump, a plunger, or the like can be used.

Step A is a step of positioning the suction tip 1. It is assumed that a cell C to be transferred is cultured in a cell culture solution Lm1 stored in a first container D1 as a movement source. The suction tip 1 is moved directly above the cell C to be sucked. Specifically, alignment is performed such that the suction port 33T and the target cell C are coaxially arranged.

Step B is a step of causing the suction tip 1 to suck the cell C. The suction tip 1 is lowered as a whole to cause the suction port 33T to be inserted into the cell culture solution Lm1 of the first container D1. At this time, the suction port 33T is caused to be as close as possible to the target cell C. Then, the negative pressure supply mechanism 100 is caused to generate a negative pressure to generate a suction force at the suction port 33T. By this operation, the cell C and a part of the cell culture solution Lm1 are sucked into the suction path 1H of the suction tip 1. Step C is a step of transferring the cell C. The suction tip 1 is moved to a second container D2 as a movement destination in a state where the cell C sucked into the suction path 1H is held.

Step D is a step of causing the suction tip 1 to discharge the cell C. The suction tip 1 is lowered to cause the suction port 33T to approach a cell culture solution Lm2 of the second container D2 or to be inserted into the cell culture solution Lm2. Then, the negative pressure supply mechanism 100 is caused to generate a positive pressure to generate a discharge force at the suction port 33T. By this operation, the cell C held in the suction path 1H of the suction tip 1 is discharged into the cell culture solution Lm2 of the second container D2. By performing the above Steps A to D, the cell C is moved from the first container D1 to the second container D2.

### [Method of Externally Fitting Sub-Tip to Base Tip]

Figs. 6A to 6C are cross-sectional views illustrating an example of a procedure of externally fitting the sub-tip 3 to the base tip 2. In this example, an example using a mounting jig 7 will be described. The mounting jig 7 has a guide hole 70 into which the sub-tip 3 and the base tip 2 are inserted. The guide hole 70 includes a first hole 71 on the +Z side (upper side) and a second hole 72 having an inner diameter smaller than that of the first hole 71. At a boundary between the first hole 71 and the second hole 72, a first locking part 73 is provided, which is a stepped portion based on a difference in an inner diameter between the first hole 71 and the second hole 72.

The inner diameter of the first hole 71 is slightly larger than the first proximal end portion 21 of the base tip 2. The inner diameter of the second hole 72 is slightly smaller than the first proximal end portion 21, and is larger than the second proximal end portion 31 of the sub-tip 3. Therefore, when the base tip 2 is inserted from the +Z end of the guide hole 70 into the first distal end portion 23 side, the first flange part 25 is locked to the first locking part 73. On the other hand, when inserted into the guide hole 70, the sub-tip 3 passes through the first locking part 73 and reaches the second hole 72. The second hole 72 has a length in the Z direction set to be longer than that of the first main body portion 22 of the base tip 2.

A receiving cylinder 74 is inserted near the -Z end of the guide hole 70. The receiving cylinder 74 is smaller in diameter than the second proximal end portion 31 of the sub-tip 3 and has a hollow portion 74H having a circular cross section capable of receiving the second main body portion 32. A second locking part 75, which is a stepped portion based on a difference in an inner diameter between the second hole 72 and the hollow portion 74H, is provided at a boundary between the second hole and the hollow portion. In the sub-tip 3 inserted into the guide hole 70, the second flange part 34 is locked at the second locking part 75.

A procedure of the external fitting will be described. First, as illustrated in Fig. 6A, the sub-tip 3 is inserted into the guide hole 70 to cause the second flange part 34 to abut the second locking part 75 and to be positioned. With the receiving cylinder 74 set to be detachably attached to the guide hole 70 and with the sub-tip 3 set in the receiving cylinder 74, the receiving cylinder 74 may be inserted from the -Z end of the guide hole 70.

Next, as illustrated in Fig. 6B, the base tip 2 is inserted from the +Z end of the guide hole 70. Since the first proximal end portion 21 of the base tip 2 is guided by an inner wall of the first hole 71, the base tip 2 is allowed to descend in the guide hole 70 with an approximately vertical attitude. Therefore, the first distal end portion 23 can be inserted into the guide passage 3H of the sub-tip 3. Even if the vertical attitude of the base tip 2 is disturbed a little, the first distal end portion 23 is led by the leading tapered surface 35 and is allowed to enter the guide passage 3H. Accordingly, it is possible to prevent the first distal end portion 23 from colliding with a pipe wall of the sub-tip 3 and deforming and breaking.

Lastly, as illustrated in Fig. 6C, the base tip 2 is pressed using a pressing rod 76 to press the first distal end portion 23 into the guide passage 3H by a predetermined depth, so that the sub-tip 3 is externally fitted to the base tip 2. The pressing rod 76 enters the hollow portion 21H (see Fig. 2) of the first proximal end portion 21 and presses a +Z-side end surface of the tapered cylinder portion 24. As the pressing proceeds, the first flange part 25 of the base tip 2 eventually abuts the first locking part 73, so that the first distal end portion 23 is inserted into the guide passage 3H at a constant pressing depth.

### [Cell Transfer Device to Which Suction Tip Is Applied]

Subsequently, a cell transfer device 5 (biological subject transfer device) to which the suction tip 1 according to the present embodiment is suitably applied will be exemplified. Fig. 7 is a perspective view showing a configuration example of the cell transfer device 5. The cell transfer device 5 includes a cell transfer line 50, a head unit 61, a lighting unit 62, and an imaging unit 63. In Fig. 7, illustration of a base supporting the cell transfer line 50 and a transfer mechanism for each unit is omitted.

The head unit 61 includes a plurality of the heads 10 to which the above suction tip 1 is attached. The head 10 is provided with a mechanism that causes a suction force and a discharge force to be generated at the suction port 33T of the attached suction tip 1. The head unit 61 is movable in an X direction and a Y direction, and can move along a predetermined movement path on the cell transfer line 50. The head 10 can move up and down in the Z direction.

The cell transfer line 50 is configured with elements arrayed in the X direction, the elements being necessary for performing a series of cell transfer steps of picking a cell contained in a container (dish 64) of a transfer source and transferring the cell to a container (microplate 65) of a movement destination. In the cell transfer line 50, a dispensing tip stock part 52, a subject stock part 51, a tip stock part 54, a tip imaging part 55, a cell sorting part 53, a black cover placement part 57, a cell transfer part 56, and a tip disposal part 58 are arranged in a line in this order from a -X end.

The subject stock part 51 is a part that stores a cell culture solution in which a large amount of cells are dispersed, the part serving as a dispensing source. The subject stock part 51 includes a tube 511 made of a cylindrical container with an open upper surface. A cell culture solution containing cells is stored in the tube 511. The dispensing tip stock part 52 is a part for keeping a plurality of dispensing tips 15. The dispensing tip stock part 52 is provided with a holder 521 that holds the dispensing tips 15 arranged in a matrix in an erected state.

The cell sorting part 53 is a part for sorting cells of a desired size from a cell culture solution containing cells of various sizes. The cell sorting part 53 includes the dish 64 that stores a cell culture solution, a holding table 531 that positions and holds the dish 64, and a table lid member 532 that covers an upper surface of the dish 64. The dish 64 includes a plate having a plurality of recesses for carrying cells on the upper surface side. Here, the dish 64 serves as a container for holding the cell of the movement source. An image of the cell in a state of being carried in the recess is captured by the imaging unit 63 under lighting of the lighting unit 62. As a result, a position of the cell to be sucked is specified.

The tip stock part 54 (stock part) includes a holding box 541 that holds a large number of the above-described suction tips 1 arranged in a matrix. The suction tip 1 can be attached to and detached from the head 10 of the head unit 61. The suction tip 1 has a function of sucking a cell carried by the dish 64, transporting the cell as the head unit 61 moves, and discharging the cell to the cell transfer part 56.

In the holding box 541, the base tip 2 to which the sub-tip 3 is attached in advance is stocked in a state where the tubular passage 2H faces upward. In other words, the suction tip 1 is held in the holding box 541 in a state where the suction tip can be easily attached to the head 10 moving in the Z direction. Note that the base tip 2 and the sub-tip 3 may be separately stocked, and after the base tip 2 is first attached to the head 10, the sub-tip 3 may be further attached using the mounting jig 7 illustrated in Fig. 6. In the tip stock part 54, a reservoir 542 for storing an impregnation liquid for wetting the suction port 33T of the suction tip 1 is also arranged.

The tip imaging part 55 is a pit that provides a position where an image of the suction tip 1 attached to the head 10 is captured. The imaging is performed by the imaging unit 63. An XYZ coordinate position of the suction port 33T of the suction tip 1 is obtained on the basis of the image of the suction tip 1 and focal position information at the time of imaging. A correction value is derived from a difference between the coordinate position and a predetermined reference position, and is used as a correction value at the time of movement control of the head 10.

The cell transfer part 56 is a part as a transfer destination to which cells sucked from the dish 64 of the cell sorting part 53 by the suction tip 1 are transferred. The cell transfer part 56 includes the microplate 65 and a holding table 561 that positions and holds the microplate 65. The microplate 65 is a plate in which a large number of small wells 66 having open upper surfaces are arranged in a matrix. The cells held in the suction tip 1 are discharged to these wells 66.

The black cover placement part 57 is a part on which a first black cover 571 covering the cell transfer part 56 and a second black cover 572 covering the cell sorting part 53 are placed. The first and second black covers 571 and 572 are used to image cell aggregates carried on the dish 64 or the microplate 65 in a light-shielded state for observation of cell fluorescence. The tip disposal part 58 is a part where the suction tip 1 and the dispensing tip 15 after use, which have finished the suction and discharge operations of the cells, are disposed.

The lighting unit 62 is movably arranged above the cell transfer line 50 to exclusively illuminate the cell sorting part 53 and the cell transfer part 56 from above. The lighting is used as transmitted lighting when the cell held in the cell sorting part 53 or the cell transfer part 56 is imaged by the imaging unit 63. The lighting unit 62 is movable in the X direction and the Y direction.

The imaging unit 63 is arranged to be movable in an XY direction below the cell transfer line 50 in order to image the cells held by the cell sorting part 53 and the cell transfer part 56 from below. In the present embodiment, the imaging unit 63 is also used to observe a state of attachment of the suction tip 1 to the head 10 at the tip imaging part 55.

Fig. 8 is a cross-sectional view showing one example of a mechanism that causes a suction force and a discharge force to be generated at the suction port 33T of the suction tip 1. The mechanism includes the head 10 and the plunger 4 attached to the head 10. The plunger 4 is slidably accommodated in the suction path 1H of the suction tip 1, and moves forward and backward in the suction path 1H in order to generate a negative pressure and a positive pressure at the suction port 33T. The plunger 4 includes a plunger proximal end portion 41 made of a cylindrical body, a needle-shaped plunger main body portion 42, and a hemispherical portion 43 connecting the plunger proximal end portion 41 and the plunger main body portion 42.

The plunger proximal end portion 41 has an outer diameter set to be smaller than an inner diameter of the hollow portion 21H of the base tip 2 by a predetermined length. The plunger main body portion 42 has an outer diameter set slightly smaller than an inner diameter of the suction path 1H. The hemispherical portion 43 has an outer peripheral surface whose curved surface shape matches a shape of an inner peripheral surface of the tapered cylinder portion 24. The plunger 4 is assembled to the suction tip 1 in such a manner that the plunger proximal end portion 41 is accommodated in the hollow portion 21H and the plunger main body portion 42 is inserted into the suction path 1H. Fig. 8 illustrates a state in which the plunger main body portion 42 is inserted most deeply through the suction tip 1, and a plunger distal end portion 44 protrudes from the distal end opening 23T of the base tip 2.

The plunger 4 can move in the +Z direction with respect to the suction tip 1 from the state shown in Fig. 8. When the plunger 4 moves in the +Z direction by a predetermined length, the plunger distal end portion 44 retracts into the tubular passage 2H of the base tip 2. At this time, a suction force can be generated at the suction port 33T to suck a liquid containing cells around the suction port 33T into the suction path 1H. After this suction, when the plunger 4 is moved in the -Z direction, the liquid sucked into the suction path 1H can be discharged from the suction port 33T.

The head 10, with the suction tip 1 attached to a -Z end of the head, causes the suction tip 1 to perform suction and discharge operations of a subject. The head 10 includes a first tubular rod 11 that moves in the Z direction, an immovable second tubular rod 12 that accommodates the first tubular rod 11 so as to be movable in the Z direction, and a discharge rod 13 accommodated in the first tubular rod 11. The discharge rod 13 is also movable in the Z direction independently of the first tubular rod 11.

The plunger proximal end portion 41 is provided with an attachment hole 41H formed of a cylindrical hollow space having an opening on an end surface in the +Z direction. A -Z end portion of the discharge rod 13 is press-fitted into the attachment hole 41H. A +Z end surface of the plunger proximal end portion 41 is opposed to a -Z end surface of the first tubular rod 11. A -Z end portion of the immovable second tubular rod 12 is press-fitted into the hollow portion 21H provided in the first proximal end portion 21 of the base tip 2. The plunger 4 moves forward and backward by the movement of the discharge rod 13 in the Z direction, thereby causing the suction tip 1 to execute the above-described suction and discharge of the liquid. In addition, by moving the first tubular rod 11 in the -Z direction after use of the suction tip 1, the plunger 4 can be pushed out from the discharge rod 13, and at the same time, the suction tip 1 can be removed from the second tubular rod 12.

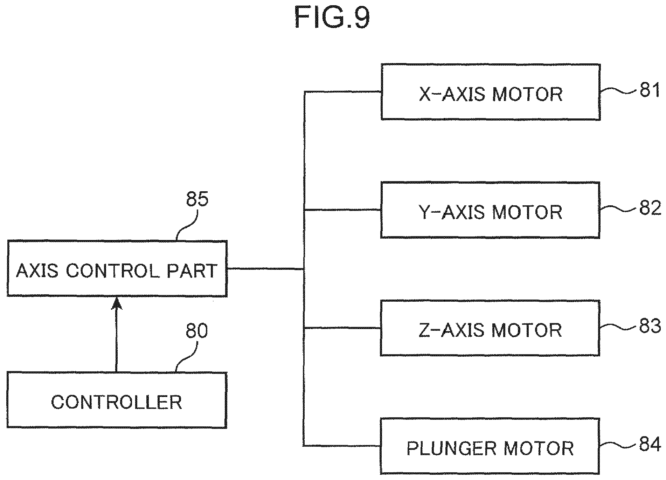

Fig. 9 is a block diagram showing an electrical configuration of the cell transfer device 5. The cell transfer device 5 includes an X-axis motor 81, a Y-axis motor 82, a Z-axis motor 83, a plunger motor 84, an axis control part 85, and a controller 80 as a transfer mechanism that causes the head 10 to perform horizontal movement and up and down movement.

The X-axis motor 81 and the Y-axis motor 82 are motors that move the head unit 61 having the head 10 in the X direction and the Y direction, respectively. The Z-axis motor 83 is a motor that moves the head 10 up and down (moves in the Z direction) as a whole. The plunger motor 84 is a motor that moves the plunger 4 in the Z direction via the discharge rod 13 and moves the first tubular rod 11 in the Z direction. The axis control part 85 drives the X-axis motor 81, the Y-axis motor 82, the Z-axis motor 83, and the plunger motor 84.

The controller 80 gives a command to the axis control part 85 to drive the X-axis motor 81 and the Y-axis motor 82, and controls the movement of the head unit 61 in the X direction and the Y direction. The controller 80 drives the Z-axis motor 83 to control the movement of the head 10 in the Z direction. The movement of the head 10 in the Z direction realizes approaching operation of the suction tip 1 to the dish 64, the microplate 65, or the like, and attaching operation of the suction tip 1 to the head 10. Further, the controller 80 controls the plunger motor 84 via the axis control part 85 to control operation of sucking and discharging the cells to the suction tip 1 and operation of releasing the suction tip 1 from the head 10.

Roughly said, the controller 80 causes the cell transfer device 5 to execute dispensing operation using the dispensing tip 15 and cell transferring operation using the suction tip 1. The controller 80 sequentially executes the following control 1 to 4 in the dispensing operation.
[Control 1] The head unit 61 is moved onto the dispensing tip stock part 52 to attach the dispensing tip 15 to a dispensing nozzle (not illustrated) mounted on the head unit 61.
[Control 2] The head unit 61 is moved onto the subject stock part 51 to cause the dispensing tip 15 to suck the cell culture solution containing cell aggregates stored in the tube 511 by a predetermined dispensing amount.
[Control 3] The head unit 61 is moved onto the cell sorting part 53 to cause the cell culture solution in the dispensing tip 15 to be discharged to the dish 64.
[Control 4] The head unit 61 is moved onto the tip disposal part 58 to remove the used dispensing tip 15 from the dispensing nozzle and dispose the same in the disposal part 58.
   The controller 80 sequentially executes the following control 5 to 8 in the cell transferring operation.
[Control 5] The head unit 61 is moved onto the tip stock part 54 to externally fit and attach, to a distal end portion of the head 10, the base tip 2 (suction tip 1) to which the sub-tip 3 is attached.
[Control 6] The head unit 61 is moved onto the cell sorting part 53 to cause the cells stored in the dish 64 to be sucked into the suction tip 1.
[Control 7] The head unit 61 is moved onto the cell transfer part 56 to cause the cells in the suction tip 1 to be discharged to the microplate 65.
[Control 8] The head unit 61 is moved onto the tip disposal part 58 to remove the used suction tip 1 from the head 10 and dispose the same in the tip disposal part 58.

According to the cell transfer device 5 described above, the suction tip 1 capable of sucking a cell of a microsize on the order of 10 µm is attached to the head 10. Accordingly, the cell transfer device 5 can suck a cell of a microsize from the dish 64, transfer the cell to the place of the microplate 65, and discharge the cell. In addition, the base tip 2 to which the sub-tip 3 is attached in advance is stocked in the tip stock part 54 in a state where the tubular passage 2H faces upward, and the base tip 2 is externally fitted to the head 10 at the tip stock part 54 as in the "control 5". Therefore, even if the suction tip 1 is of a two-split type having the base tip 2 and the sub-tip 3, the suction tip 1 can be easily attached to the head 10 as a result of stopover of the head 10 at the tip stock part 54.

### [Summary of Present Disclosure]

The specific embodiment described above includes disclosure having the following configurations.

A suction tip according to one aspect of the present invention is a suction tip that sucks a biological subject, the suction tip including: a base tip including a distal end portion having a distal end opening, and a tubular passage connected to the distal end opening; and a sub-tip including a suction port that sucks the biological subject, and a guide passage having one end connected to the suction port and another end that receives the distal end portion of the base tip; in which the base tip and the sub-tip are coupled and integrated by externally fitting the other end of the guide passage to the distal end portion, the integration forming one suction path in which the tubular passage and the guide passage communicate with each other, and the suction port has a size smaller than a size of the distal end opening.

According to this suction tip, the suction tip is configured by a combination of the base tip and the sub-tip. It is difficult to manufacture, with a single member, a suction tip having a very large aspect ratio, in which a distal end portion having a suction port is extremely small with respect to a tip length. However, in the case of the two-split type suction tip, the base tip and the sub-tip can be individually manufactured within a range in which an aspect ratio does not become excessive, and coupled to each other to obtain one suction tip, resulting in facilitating manufacture thereof. In other words, it is easy to reduce a size of the suction port.

In addition, it is structured such that the other end of the guide passage of the sub-tip is externally fit to the distal end portion of the base tip. Therefore, even if one end side connected to the suction port is set to have a minimum size, the sub-tip is allowed to have the other end having a suitable size, resulting in having excellent assembly workability. Furthermore, by making the size of the suction port of the sub-tip be smaller than the size of the distal end opening of the base tip, it is possible to selectively suck a minute biological subject with high accuracy. Naturally, by reducing the size of the suction port, the distal end portion of the suction tip having the suction port can also be reduced in size.

In the above suction tip, it is desirable that the distal end portion of the base tip is a first distal end portion having a circular cross section, the suction port is an opening provided at a second distal end portion having a circular cross section of the sub-tip, and the second distal end portion has an outer diameter smaller than an outer diameter of the first distal end portion.

According to this aspect, not only the suction port of the sub-tip but also the outer diameter of the second distal end portion having the suction port is smaller than that of the first distal end portion of the base tip. Accordingly, a biological subject as a target can be sucked into the suction tip with good selectivity with respect to surrounding biological subjects existing around the target biological subject. In addition, it is possible to make it difficult for the second distal end portion to damage the surrounding biological subject during the suction operation.

In the above suction tip, it is desirable that in a state where the base tip and the sub-tip are coupled and integrated, the distal end portion has an outer peripheral surface in close contact with an inner peripheral surface of the other end of the guide passage.

According to this aspect, liquid such as culture solution sucked together with the biological subject does not enter an interface between the base tip and the sub-tip. Accordingly, all of the sucked liquid and biological subject can be held in the suction path.

In the above suction tip, it is desirable that the outer peripheral surface of the distal end portion is a first tapered surface whose outer diameter decreases toward the distal end opening, the inner peripheral surface of the guide passage is a second tapered surface whose inner diameter decreases toward the suction port, and the first tapered surface and the second tapered surface are in close contact with each other in the coupled and integrated state.

According to this aspect, since the outer peripheral surface of the distal end portion of the base tip and the inner peripheral surface of the guide passage of the sub-tip are coupled by fitting the tapered surfaces to each other, it is easier to form a close contact state and it is possible to improve close contact.

In the above suction tip, it is desirable that a leading tapered surface is continuously provided at the other end of the guide passage, the leading tapered surface leading the distal end portion to the other end at the time of the external fitting.

According to this aspect, when the sub-tip is externally fitted to the base tip, the distal end portion of the base tip can be guided by the leading tapered surface. Accordingly, at the time of the external fitting, the distal end portion can be inserted into the guide passage without requiring precise alignment therebetween.

In the above suction tip, the sub-tip may include a first stopper part capable of positioning the guide passage in an extending direction.

According to this aspect, the sub-tip can be externally fitted to the base tip in a state where the first stopper part is held by any jig. Therefore, workability of the external fitting is improved.

In this case, it is desirable that the base tip includes a second stopper part capable of positioning the tubular passage in an extending direction.

According to this aspect, the second stopper part can be used as a part for positioning an external fitting depth of the distal end portion of the base tip. For example, at the insertion of the distal end portion of the base tip into the guide passage of the sub-tip being positioned by the first stopper part, some kind of jig against which the second stopper part abuts is arranged at a position corresponding to a required insertion depth. As a result, the sub-tip can be externally fitted to the base tip with good reproducibility.

In the above suction tip, it is desirable that the base tip and the sub-tip are both made of resin, and the distal end portion of the base tip is an insertion fitting part that is inserted into the other end of the guide passage without interposing an adhesive.

According to this aspect, due to elasticity of resin, damage at the time of assembling the suction tip or at the time of suction operation can be suppressed as compared with a case of using an easily breakable material such as glass. In addition, in the externally fitted state, while bonding properties of the sub-tip to the base tip can be secured without using an adhesive, the sub-tip can be easily removed from the base tip.

In this case, the base tip and the sub-tip are desirably made of resin of the same material.

According to this aspect, affinity between the base tip and the sub-tip can be improved. In addition, it is easy to make molding conditions, toxicity evaluation, and the like of both the tips be uniform, which can contribute to facilitation of manufacture of the suction tip.

The above suction tip may further include a plunger that is slidably accommodated in the tubular passage of the base tip and generates a negative pressure at the suction port. According to this aspect, a negative pressure can be generated at the suction port with a simple mechanism for moving the plunger forward and backward.

A biological subject transfer device according to another aspect of the present invention includes: a head to which the above suction tip is attached and which includes a mechanism that generates a suction force and a discharge force at the suction port; and a transfer mechanism that moves the head horizontally and moves the head up and down.

According to this biological subject transfer device, a suction tip capable of sucking a biological subject of a microsize is attached to the head. Accordingly, it is possible to provide a biological subject transfer device that sucks a biological subject of a microsize, transfers the biological subject to a required place, and discharges the same.

The above-described biological subject transfer device may further include: a stock part that stocks, in a state where the tubular passage faces upward, the base tip to which the sub-tip is attached; and a controller that controls the transfer mechanism so as to externally fit, at the stock part, the base tip to a distal end portion of the head.

According to this aspect, even if the suction tip is of a two-split type having the base tip and the sub-tip, the suction tip can be easily attached to the head as a result of stopover of the head at the stock part.

According to the present invention described above, it is possible to provide a suction tip suitable for sucking a microsized biological subject and easy to manufacture, and a biological subject transfer device using the suction tip.

This application is based on Japanese Patent application No. 2022-079001 filed in Japan Patent Office on May 12, 2022, the contents of which are hereby incorporated by reference.

Although the present invention has been fully described by way of example with reference to the accompanying drawings, it is to be understood that various changes and modifications will be apparent to those skilled in the art. Therefore, unless otherwise such changes and modifications depart from the scope of the present invention hereinafter defined, they should be construed as being included therein.

## Claims

1. A suction tip (1) configured to suck a biological subject (C), the suction tip (1) comprising:
a base tip (2) including a distal end portion (23) having a distal end opening (23T), and a tubular passage (2H) connected to the distal end opening (23T); and
a sub-tip (3) including a suction port (33T) configured to suck the biological subject (C), and a guide passage (3H) having one end (3HA) connected to the suction port (33T) and another end (3HB) that receives the distal end portion (23) of the base tip (2); wherein
the base tip (2) and the sub-tip (3) are coupled and integrated by externally fitting the other end (3HB) of the guide passage (3H) to the distal end portion (23), the integration forming one suction path (1H) in which the tubular passage (2H) and the guide passage (3H) communicate with each other, and
the suction port (33T) has a size smaller than a size of the distal end opening (23T).

2. The suction tip (1) according to claim 1, wherein
the distal end portion of the base tip (2) is a first distal end portion (23) having a circular cross section,
the suction port (33T) is an opening provided at a second distal end portion (33) having a circular cross section of the sub-tip (3), and
the second distal end portion (33) has an outer diameter smaller than an outer diameter of the first distal end portion (23).

3. The suction tip (1) according to claim 1 or 2, wherein
in a state where the base tip (2) and the sub-tip (3) are coupled and integrated, the distal end portion (23) has an outer peripheral surface in close contact with an inner peripheral surface of the other end (3HB) of the guide passage (3H).

4. The suction tip (1) according to claim 3, wherein
the outer peripheral surface of the distal end portion (23) is a first tapered surface (T1) whose outer diameter decreases toward the distal end opening (23T),
the inner peripheral surface of the guide passage (3H) is a second tapered surface (T2) whose inner diameter decreases toward the suction port (33T), and
the first tapered surface (T1) and the second tapered surface (T2) are in close contact with each other in the coupled and integrated state.

5. The suction tip according to any one of claims 1 to 4, wherein
a leading tapered surface (35) is continuously provided at the other end (3HB) of the guide passage (3H), the leading tapered surface (35) leading the distal end portion (23) to the other end (3HB) at the time of the external fitting.

6. The suction tip (1) according to any one of claims 1 to 5, wherein
the sub-tip (3) includes a first stopper part (34) capable of positioning the guide passage (3H) in an extending direction.

7. The suction tip (1) according to claim 6, wherein
the base tip (2) includes a second stopper part (25) capable of positioning the tubular passage (2H) in an extending direction.

8. The suction tip (1) according to any one of claims 1 to 7, wherein
the base tip (2) and the sub-tip (3) are both made of resin, and
the distal end portion (23) of the base tip (2) is an insertion fitting part that is inserted into the other end (3HB) of the guide passage (3H) without interposing an adhesive.

9. The suction tip (1) according to claim 8, wherein
the base tip (2) and the sub-tip (3) are made of resin of a same material.

10. The suction tip (1) according to any one of claims 1 to 9, further comprising:
a plunger (4) that is slidably accommodated in the tubular passage (2H) of the base tip (2) and generates a negative pressure at the suction port (33T).

11. A biological subject transfer device (5) comprising:
a head (10) to which the suction tip (1) according to any one of claims 1 to 10 is attached and which includes a mechanism configured to generate a suction force and a discharge force at the suction port (33T); and
a transfer mechanism (81,82,83) configured to move the head (10) horizontally and moves the head (10) up and down.

12. The biological subject transfer device (5) according to claim 11, further comprising:
a stock part (54) configured to stock, in a state where the tubular passage (2H) faces upward, the base tip (2) to which the sub-tip (3) is attached; and
a controller (80) configured to control the transfer mechanism so as to externally fit, at the stock part (54), the base tip (2) to a distal end portion of the head (10).
